# FASCICULE DE BREVET EUROPEEN

(11) **EP 1 861 059 B1**
(45) Date de publication et mention de la délivrance du brevet: **05.11.2008**
(21) Numéro de dépôt: 06726125.5
(22) Date de dépôt: 22.03.2006
(51) Int. Cl.: A61F 15/00

(54) **NECESSAIRE ET PROCEDE POUR LA PROTECTION DE PANSEMENTS ET/OU DE ZONES CUTANEES BLESSEES DU CORPS**
KIT UND VERFAHREN ZUM SCHUTZ VON VERBÄNDEN UND/ODER VERLETZTEN HAUTREGIONEN AM KÖRPER
KIT AND METHOD FOR PROTECTING DRESSINGS AND/OR WOUNDED SKIN REGIONS OF THE BODY

(30) Priorité: 23.03.2005 FR 0502847
(43) Date de publication de la demande: 05.12.2007
(73) Titulaire: Douchossec, 13704 La Ciotat Cedex (FR)
(72) Inventeur: Gilli, Eric, 13009 Marseille (FR)
(74) Mandataire: Marek, Pierre
(86) Numéro de dépôt international: PCT/FR2006/000627
(87) Numéro de publication internationale: WO 2006/100382

(56) Documents cités:
- WO-A-94/23677
- US-A- 5 599 290
- US-A1- 2003 212 358

## Description

La présente invention concerne un nécessaire pour la protection de pansements et/ou de zones cutanées blessées du corps.

Différents dispositifs ont été proposés à ce jour, pour assurer la protection des pansements appliqués sur des zones cutanées blessées du corps ou directement sur ces zones, afin d'éviter que ces pansements ou blessures ne soient mouillés ou souillés par des projections d'eau ou autres liquides pollués. Par exemple, ces dispositifs sont prévus pour que des personnes portant des pansements puissent se baigner ou se doucher, sans crainte de les mouiller.

Les dispositifs de l'état de la technique sont constitués par des feuilles souples dédiées, généralement rectangulaires (WO-89/03765, WO-99/02110), ou par des gaines (WO-91/17733, WO-94/24971, FR-2 706 290), ou par des manchons (EP-0 358 451, WO-93/14730, FR-2 686 786) munis, à demeure et d'origine, de moyens permettant leur fixation amovible sur les zones à protéger.

Un inconvénient commun à tous ces dispositifs, plus précisément sensible pour les particuliers, résulte du fait que les dimensions, formes et emplacements des surfaces couvertes par les pansements ou les lésions, sont imprévisibles et très variables, de sorte que les utilisateurs potentiels sont conduits à acheter et à conserver une large gamme de tailles de dispositifs de protection pour couvrir un éventail intéressant de surfaces. Il en résulte que l'acheteur disposera d'un certain nombre d'articles soit trop petits, soit trop grands, qui ne seront jamais utilisés et qu'il faudra jeter après un certain temps de conservation. En outre, la forme de ces articles n'est pas adaptable à la forme des pansements ou à celle des surfaces cutanées traumatisées à protéger, de sorte qu'elle peut être une source de gêne dans les mouvements du membre ou de la partie du corps portant le pansement.

Pour résoudre un problème différent, on connaît (WO-94/23677) une couverture thermique utilisable pour prévenir une perte de chaleur des parties du corps humain ou animal, constituée d'une feuille de métal réfléchissant ou d'un film de matière plastique métallisée munie de perforations permettant des échanges gazeux et se fixant sur la peau au moyen d'un ruban adhésif, de préférence double face et également muni de perforations. Un tel dispositif ne peut être utilisé pour assurer une protection efficace des zones cutanées traumatisées du corps contre les risques de souillure ou de contamination par contact avec des éléments impurs, notamment avec des liquides pollués, non seulement en raison du fait que la feuille de couverture est pourvue de perforations mais surtout parce que l'emploi d'un ruban double face, muni ou non de perforations, pour réaliser la fixation de ladite feuille sur la peau, ne peut garantir l'étanchéité entre celles-ci, surtout lorsque les zones couvertes présentent des formes irrégulières et/ou mouvantes.

Un objet de l'invention est de remédier aux inconvénients des dispositifs de protection proposés à ce jour.

Selon l'invention, ce but est atteint grâce à un nécessaire de protection comprenant une trousse renfermant :
- d'une part, au moins une feuille globale ou feuille de base non dédiée, mince, souple et étanche à l'eau, de dimensions permettant la découpe d'une pluralité de pièces de couverture singulières ou individuelles adaptées, cette feuille globale étant, de préférence, exécutée dans un matériau non allergénique et facilement divisible en morceaux ou fractions de dimensions plus réduites, par exemple au moyen d'une paire de ciseaux, et,
- d'autre part, un conditionnement, par exemple constitué par un tube compressible, renfermant un adhésif pâteux, étanche à l'eau et non allergénique, et, de préférence, muni d'un goulot de distribution de cet adhésif sous forme de cordon, ledit adhésif présentant des propriétés lui permettant d'adhérer à la fois à la peau et à la feuille de protection.

On comprend que le nécessaire de protection selon l'invention permet la réalisation d'une protection "sur mesure" des pansements ou des zones cutanées traumatisées du corps (écorchures, cicatrices, piqûres d'insectes, coups de soleil,...) dont l'apparition est généralement imprévue et qui peuvent concerner des parties du corps de conformations très variées. En outre, la protection assurée par le dispositif de l'invention procure une garantie contre les risques de souillure ou de contamination des parties protégées, par contact avec des éléments impurs, y compris avec des liquides pollués.

En effet, le dépôt d'un cordon continu d'adhésif entre la peau et la feuille de protection permet non seulement une fixation efficace de celle-ci, mais également d'assurer la fonction de barrière parfaitement étanche. Cette barrière continue placée en liaison directe entre la peau et la feuille permet de garantir une fixation périphérique totale, sans solution de continuité, et une excellente étanchéité.

Le nécessaire de protection selon l'invention est, de plus, d'utilisation facile et rapide. Il est également très économique.

Les buts, caractéristiques et avantages ci-dessus, et d'autres encore, ressortiront mieux de la description qui suit et des dessins annexés dans lesquels:

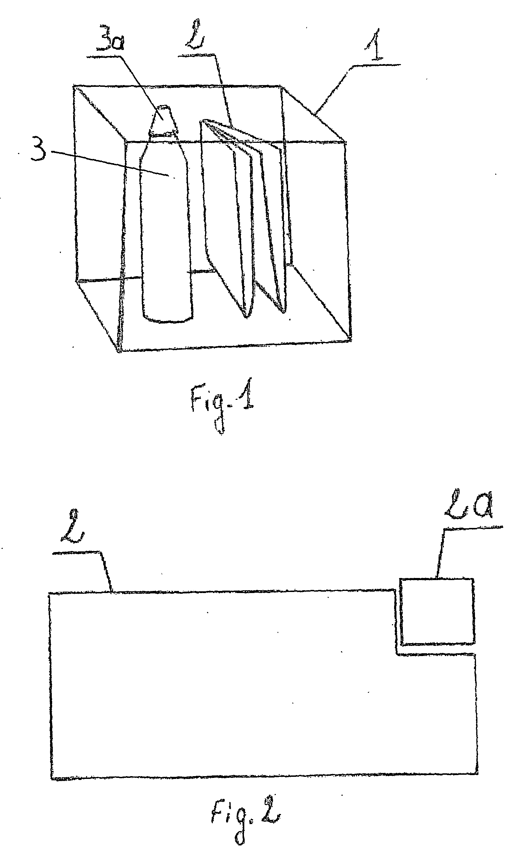

La figure 1 est une vue en perspective, à caractère schématique, d'un nécessaire de protection selon l'invention.
La figure 2 est une vue en plan montrant le découpage d'une portion de feuille de couverture individuelle adaptée, à partir d'une feuille de base non dédiée.

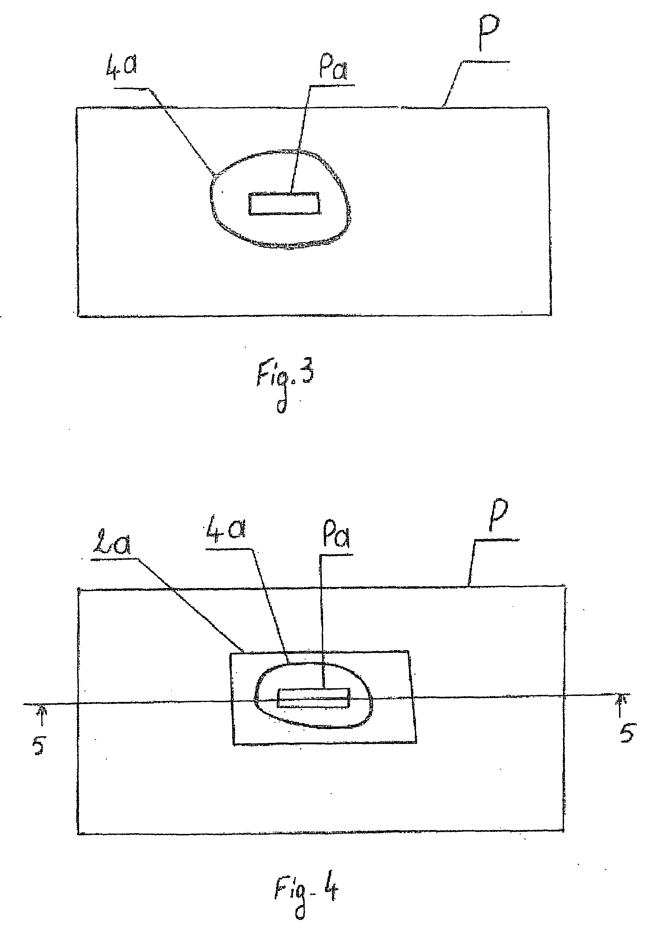

La figure 3 est une vue en plan et à plus grande échelle illustrant l'application d'un cordon d'adhésif sur une zone cutanée, autour d'un pansement.
La figure 4 est une vue en plan montrant l'application de la feuille de protection découpée sur le cordon d'adhésif déposé, en couverture du pansement.

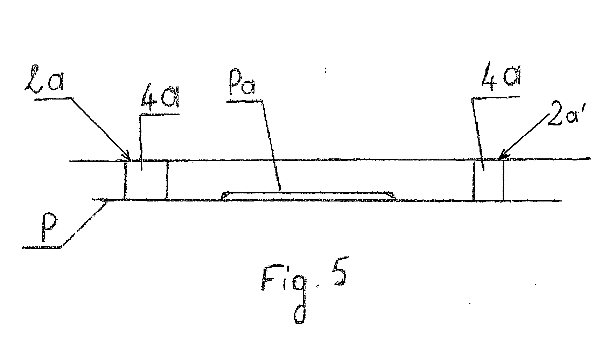

La figure 5 est une vue en coupe et à plus grande échelle selon la ligne 5-5 de la figure 4.

On se reporte auxdits dessins pour décrire un exemple intéressant de réalisation du nécessaire de protection selon l'invention.

Le nécessaire de protection selon l'invention comprend essentiellement :
- une pochette 1 ;
- au moins une feuille de base ou feuille globale souple 2 non dédiée ;
- un récipient d'adhésif 3.

La pochette 1 peut être exécutée en toute matière, toute forme et toutes dimensions appropriées, le mot "pochette" n'ayant, par ailleurs, aucun caractère restrictif, ce terme pouvant désigner tout article analogue tel que : trousse, sac, étui, mallette, boîte, etc. D'autre part, la pochette 1 peut être munie de tout système de fermeture adéquat (fermeture à glissière, à bouton-pression, etc).

La feuille de base ou feuille globale non dédiée 2 peut être exécutée en toute matière mince et souple et, de préférence, dans un matériau non allergénique, par exemple en polyuréthanne ou en polymère à base de polyuréthanne, étanche à l'eau et facilement découpable au moyen d'un instrument manuel tel qu'une paire de ciseaux. Elle présente des dimensions permettant le découpage d'une pluralité de parties unitaires spécifiques 2a appropriées à la couverture de pansements ou blessures de dimensions, formes et situations variées. Par exemple, elle peut présenter une surface comprise entre 1600 cm² et 2500 cm². Elle peut être transparente, translucide, opaque, blanche ou teintée, voire ornée de motifs divers.

D'autre part, elle présente, de manière avantageuse, une épaisseur inférieure à 500 µm, par exemple une épaisseur comprise entre 5 µm et 500 µm et, de préférence, une épaisseur comprise entre 10 µm et 80 µm.

Le récipient d'adhésif 3 peut être constitué par un tube compressible contenant un adhésif de consistance pâteuse 4. L'adhésif contenu dans le récipient est étanche à l'eau et possède des propriétés qui lui permettent d'adhérer à la fois à la peau et à la feuille de protection plastique, sans avoir été collé auparavant à l'une ou l'autre des deux surfaces. Bien entendu, tout autre type de récipient serait utilisable.

L'adhésif 4 est également non allergénique et biocompatible. Il peut être avantageusement constitué par un copolymère, de préférence à base de silicone, par exemple par une colle à base de polydiméthylsiloxane, avec groupes fonctionnels.

Le récipient 3 comporte, de préférence, une tête d'extrusion ou un goulot 3a agencé, de manière connue en soi, pour permettre la distribution de l'adhésif 4 contenu dans ledit récipient sous forme d'un cordon souple 4a.

Selon le mode de mise en oeuvre du nécessaire de protection de l'invention, une fraction de feuille 2a est découpée aux dimensions et formes souhaitables, en fonction de la taille et de la forme du pansement ou de la zone blessée de la peau P, dans une feuille de base ou feuille globale non dédiée 2, mince, souple et étanche à l'eau, de préférence réalisée dans un matériau non allergénique, et de dimensions permettant la découpe d'une pluralité de fractions ou portions individuelles de protection, un cordon 4a d'adhésif souple également étanche à l'eau et non allergénique, et présentant en outre des propriétés lui permettant d'adhérer à la fois à la peau et à la feuille de protection, est déposé sur la peau P, autour du pansement Pa ou de la zone cutanée traumatisée à protéger, et ladite portion ou fraction de feuille individuelle adaptée est enfin disposée en couverture du pansement ou de la zone cutanée blessée, de sorte que sa bordure 2a' vienne s'appliquer sur le cordon périphérique d'adhésif 4a, en réalisant ainsi une jonction intime entre la peau et la feuille de couverture individuelle adaptée et l'isolement du pansement et/ou de la zone cutanée blessée.

## Revendications

1. Nécessaire pour la protection de pansements et/ou de zones cutanées traumatisées du corps, **caractérisé en ce qu'**il comprend une trousse (1) renfermant :
- d'une part, au moins une feuille de base ou feuille globale non dédiée (2), souple et étanche à l'eau, de dimensions permettant la découpe d'une pluralité de pièces de couvertures singulières ou individuelles adaptées (2a), cette feuille de base (2) étant, de préférence, exécutée dans un matériau non allergénique et, facilement divisible en morceaux ou fractions de dimensions plus réduites, par exemple au moyen d'une paire de ciseaux et,
- d'autre part, un conditionnement (3), renfermant un adhésif pâteux, étanche à l'eau et non allergénique, ledit adhésif présentant des propriétés lui permettant d'adhérer à la fois à la peau et à la feuille de protection.

2. Nécessaire pour la protection de pansements et/ou de zones cutanées traumatisées du corps, selon la revendication 1, **caractérisé en ce que** le conditionnement (3) est constitué par un tube compressible.

3. Nécessaire pour la protection de pansements et/ou de zones cutanées traumatisées du corps, selon l'une des revendications 1 ou 2, **caractérisé en ce que** le conditionnement (3) est muni d'une tête d'extrusion ou d'un goulot (3a) de distribution de cet adhésif sous forme de cordon (4a).

4. Nécessaire pour la protection de pansements et/ou de zones cutanées traumatisées du corps, selon l'une quelconque des revendications 1 à 3, **caractérisé en ce que** la feuille de base ou feuille globale non dédiée (2) est exécutée en polyuréthanne, ou en polymère à base de polyuréthanne.

5. Nécessaire pour la protection de pansements et/ou de zones cutanées traumatisées du corps, selon l'une quelconque des revendications 1 à 4, **caractérisé en ce que** l'adhésif est constitué par un adhésif constitué par un copolymère, de préférence à base de silicone, par exemple par une colle à base de polydiméthylsiloxane.

6. Nécessaire pour la protection de pansements et/ou de zones cutanées traumatisées du corps, selon l'une quelconque des revendications 1 à 5, **caractérisé en ce que** la feuille de base ou feuille globale non dédiée (2) présente une épaisseur inférieure à 500 µm, par exemple une épaisseur comprise entre 5µm et 500 µm, de préférence une épaisseur comprise entre 10 µm et 80 µm.

## Claims

1. A kit for protecting dressings and/or traumatised skin regions on the body, **characterised in that** it comprises a case (1) containing:
- on the one hand at least one basic or global non-dedicated flexible and waterproof sheet (2) of dimensions such as to permit a plurality of suited singular or individual cover portions (2a) to be cut out, said basic sheet (2) preferably being made of a non-allergenic material easily divisible into pieces or fractions of smaller dimensions, for example by means of a pair of scissors, and
- on the other hand a packaging (3) containing a pasty, waterproof and non-allergenic adhesive, said adhesive having properties permitting it to adhere both to the skin and the protection sheet.

2. A kit for protecting dressings and/or traumatised skin regions on the body according to claim 1 **characterised in that** the packaging (3) is formed by a compressible tube.

3. A kit for protecting dressings and/or traumatised skin regions on the body according to one of claims 1 and 2 **characterised in that** the packaging (3) is provided with an extrusion head or a spout (3a) for distribution of said adhesive in the form of a bead (4a).

4. A kit for protecting dressings and/or traumatised skin regions on the body according to any one of claims 1 to 3 **characterised in that** the basic or global non-dedicated sheet (2) is made of polyurethane or polyurethane-based polymer.

5. A kit for protecting dressings and/or traumatised skin regions on the body according to any one of claims 1 to 4 **characterised in that** the adhesive is formed by an adhesive formed by a copolymer, preferably a silicone-based copolymer, for example a polydimethylsiloxane-based glue.

6. A kit for protecting dressings and/or traumatised skin regions on the body according to any one of claims 1 to 5 **characterised in that** the basic or global non-dedicated sheet (2) is of a thickness of less than 500 µm, for example a thickness of between 5 µm and 500 µm, preferably a thickness of between 10 µm and 80 µm.

## Patentansprüche

1. Set für den Schutz von Verbänden und/oder von verletzten Hautzonen des Körpers, **dadurch gekennzeichnet, dass** es eine Tasche (1) aufweist, die enthält:
- einerseits mindestens eine nicht zweckbestimmte Basisfolie oder Gesamtfolie (2), die biegsam und wasserdicht ist und Abmessungen aufweist, die das Ausschneiden mehrerer geeigneter einzelner oder individueller Abdeckungen (2a) erlauben, wobei diese Basisfolie (2) vorzugsweise aus einem anti-allergischen Werkstoff hergestellt ist und leicht in Stücke oder Teile geringerer Abmessungen aufgeteilt werden kann, zum Beispiel mittels einer Schere, und
- andererseits eine Verpackung (3), die einen pastenförmigen, wasserdichten und anti-allergischen Klebstoff enthält, wobei der Klebstoff Eigenschaften hat, die es ihm ermöglichen, sowohl an der Haut als auch an der Schutzfolie zu kleben.

2. Set für den Schutz von Verbänden und/oder von verletzten Hautzonen des Körpers nach Anspruch 1, **dadurch gekennzeichnet, dass** die Verpackung (3) aus einem zusammendrückbaren Rohr besteht.

3. Set für den Schutz von Verbänden und/oder von verletzten Hautzonen des Körpers nach einem der Ansprüche 1 oder 2, **dadurch gekennzeichnet, dass** die Verpackung (3) mit einem Strangpresskopf oder einem Flaschenhals (3a) zur Ausgabe dieses Klebstoffs in Form eines Strangs (4a) versehen ist.

4. Set für den Schutz von Verbänden und/oder von verletzten Hautzonen des Körpers nach einem der Ansprüche 1 bis 3, **dadurch gekennzeichnet, dass** die nicht zweckbestimmte Basisfolie oder Gesamtfolie (2) aus Polyurethan oder aus einem Polymer auf der Basis von Polyurethan hergestellt ist.

5. Set für den Schutz von Verbänden und/oder von verletzten Hautzonen des Körpers nach einem der Ansprüche 1 bis 4, **dadurch gekennzeichnet, dass** der Klebstoff aus einem Copolymer vorzugsweise auf der Basis von Silikon besteht, zum Beispiel aus einem Leim auf der Basis von Polydimethylsiloxan.

6. Set für den Schutz von Verbänden und/oder von verletzten Hautzonen des Körpers nach einem der Ansprüche 1 bis 5, **dadurch gekennzeichnet, dass** die nicht zweckbestimmte Basisfolie oder Gesamtfolie (2) eine Dicke von weniger als 500 µm, zum Beispiel eine Dicke zwischen 5 µm und 500 µm, vorzugsweise eine Dicke zwischen 10 µm und 80 µm, hat.
